# EUROPÄISCHE PATENTANMELDUNG

(11) **EP 1 216 671 A2**
(43) Veröffentlichungstag der Anmeldung: **26.06.2002**
(21) Anmeldenummer: 01124740.0
(22) Anmeldetag: 17.10.2001
(51) Int. Cl.: A61F 9/00

(54) **Vorrichtung zur Glaukombehandlung**

(30) Priorität: 14.12.2000 DE 10062478
(71) Anmelder: Glautec AG, 90425 Nürnberg (DE)
(72) Erfinder: Haefliger, Eduard, Dr., 4067 Basel (CH)
(74) Vertreter: Matschkur, Lindner, Blaumeier Patent- und Rechtsanwälte

(57) **Zusammenfassung**

Vorrichtung zur Glaukombehandlung mit einem Laserkatheter und einer lichtführenden Faseranordnung, an deren proximalem Ende Licht einkoppelbar ist und an deren distalem Ende eine Lichtaustrittsfläche vorgesehen ist, die bei der Behandlung einem im Schlemm'schen Kanal angeordneten gebogenen Stent gegenübersteht, wobei der Stent einen im Wesentlichen dreieckigen Querschnitt derart aufweist, dass die Lichtaustrittsfläche im Wesentlichen kongruent zur gegenüberliegenden Seitenfläche des Stents im Schlemm'schen Kanal ausgebildet ist.

## Beschreibung

Die Erfindung bezieht sich auf eine Vorrichtung zur Glaukombehandlung mit einem Laserkatheter und einer lichtführenden Faseranordnung, an deren proximalem Ende Licht einkoppelbar ist und an deren distalem Ende eine Lichtaustrittsfläche vorgesehen ist, die bei der Behandlung einem im Schlemm'schen Kanal angeordneten gebogenen Stent gegenübersteht.

Zur Glaukombehandlung, also zur Beseitigung eines Überdrucks im Augapfel, gibt es neben medikamentösen Verfahren eine Reihe von chirurgischen Verfahren. Unter anderem ist dabei auch bereits eine Vorrichtung der vorstehend beschriebenen Art vorgeschlagen worden, bei welcher mittels UV-Licht das vorzugsweise über einen Excimerlaser erzeugt und über Lichtleiter in das Augeninnere geführt wird, das schwammartige Trabekelwerk, durch das Kammerwasser aus der vorderen und hinteren Augenkammer fließt, lokal abgetragen wird, so dass das Kammerwasser leichter in den Schlemm'schen Kanal gelangen kann, durch den es schließlich abgeführt wird.

Zum Einsatz dieser Vorrichtung ist es erforderlich, das Auge lokal zu öffnen, um mit Hilfe des lichtleitenden Laserkatheters Licht in unmittelbare Nähe des zu perforierenden Gewebes des Trabekelwerkes zu leiten. Notwendig ist es dabei, die Lichtaustrittsfläche genau vor dem Schlemm'schen Kanal zu positionieren, um genau an dieser Stelle das Trabekelwerk zu durchlöchern und den Abfluss in den Schlemm'schen Kanal sicherzustellen. Diese Positionierung des distalen Endes des Laserkatheters ist aber sehr schwierig und macht dadurch den Einsatz einer solchen Glaukombehandlungsvorrichtung bis zu einem gewissen Grad zu einem Glücksspiel, bei dem es darauf ankommt, ob man die richtige Position beim Einführen des Laserkatheters wirklich gefunden hat oder nicht. Besonders schwierig ist es dabei, sicherzustellen, dass das austretende Laserlicht nicht das Gewebe seitlich neben oder hinter dem Schlemm'schen Kanal zerstört, sondern ausschließlich das davor liegende Gewebe des Trabekelwerkes. Bei Verwendung der üblicherweise zylindrischen Stents ist eine dementsprechende Positionierung und die Vermeidung von durch Reflexion seitlich abgelenktem Licht aber sehr schwierig.

Der Erfindung liegt daher die Aufgabe zugrunde, eine Vorrichtung der eingangs genannten Art derart weiterzubilden, dass in einfacher Weise eine Beschädigung des nicht betroffenen Gewebes neben und hinter dem Schlemm'schen Kanal sicher vermieden wird.

Zur Lösung dieser Aufgabe ist erfindungsgemäß vorgesehen, dass der Stent einen im Wesentlichen dreieckigen Querschnitt derart aufweist, dass die Lichtaustrittsfläche im Wesentlichen kongruent zur gegenüberliegenden Seitenfläche des Stents im Schlemm'schen Kanal ausgebildet ist.

Durch die erfindungsgemäße Anordnung lässt sich das Gewebe des Trabekelwerks beim Einführen des Laserkatheters durch die die Spitze bildende Lichtaustrittsfläche gegen die parallel dazu angeordnete Seitenfläche des Stents zusammenpressen, so dass das Laserlicht ausschließlich eine Öffnung im Gewebe vor dem Schlemm'schen Kanal ausbrennt, aber keinerlei Gefahr besteht, dass seitlich reflektiertes Licht zu einer Beschädigung des Gewebes an anderen Stellen führen kann. Ausgehend von der Lage des Schlemm'schen Kanals ergibt sich bei im Wesentlichen frontal zur Augenvorderfläche gerichteter Führung des Laserkatheters die vorstehend beschriebene besondere Parallelstellung dadurch, dass - ausgehend davon, dass die Lichtaustrittsfläche unter einem Winkel von 65° gegenüber der Achse der lichtführenden Faseranordnung geneigt ist - die auf der Bogeninnenseite liegende, der Lichtaustrittsfläche des Laserkatheters gegenüberliegende Innenfläche einen Winkel von ca. 115° mit der schmalen Basisfläche und die auf der Bogenaußenseite liegende Außenfläche einen Winkel von ca. 40° mit der Basisfläche des Stents bilden.

Der bevorzugt einen Biegeradius von ca. 7 mm - entsprechend dem Biegeradius des Schlemm'schen Kanals - aufweisende Stent, der angepasst an den Durchmesser des Schlemm'schen Kanals eine Querschnittsabmessung von ca. 0,15 mm aufweisen soll, kann in weiterer Ausgestaltung der Erfindung am einen Ende mit einem Plättchen oder einer Nocke zum Fassen mittels einer Pinzette, Zange od. dgl. versehen sein.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: eine vergrößerte perspektivische Ansicht eines erfindungsgemäßen Stents,
- Fig. 2: einen schematischen Teilschnitt durch die vordere und hintere Augenkammer mit dem Schlemm'schen Kanal mit dem eingelegten Stent und dem angedeuteten vorderen Ende eines Laserkatheters zur Glaukombehandlung,
- Fig. 3: einen vergrößerten Teilausschnitt aus Fig. 2 mit dem Stent und dem davor positionierten Ende der lichtführenden Faseranordnung des Laserkatheters und
- Fig. 4: einen im Wesentlichen der Fig. 3 entsprechenden Teilausschnitt mit einer abgewandelten, eine Justierausnehmung umfassenden Faseranordnung.

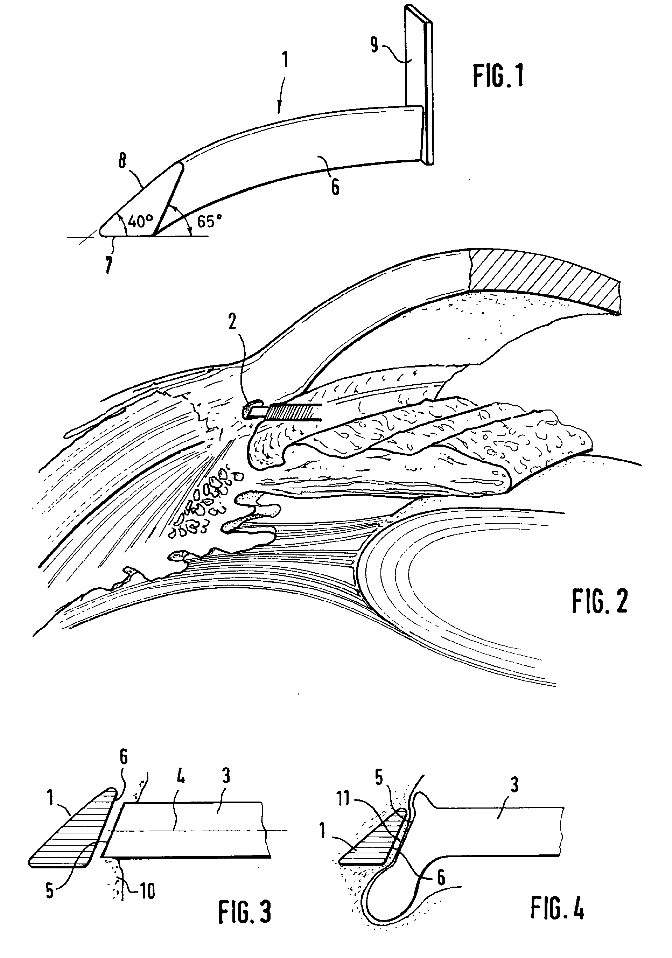

Der in Fig. 1 angedeutete, beispielsweise aus Edelstahl od. dgl. bestehende Stent 1 ist im Gegensatz zu den bisherigen Anordnungen, bei denen der Querschnitt rund ausgebildet ist, im Wesentlichen dreieckförmig im Querschnitt ausgebildet, wobei die bei der Positionierung im Schlemm'schen Kanal 2 dem Ende der lichtleitenden Faseranordnung 3 eines Laserkatheters mit der unter 65° zur Längsachse 4 geschnittenen Lichtaustrittsfläche 5 gegenüberliegende Vorderfläche 6 des Stents gegenüber der schmalen Basisfläche 7 unter einem Winkel von 115° geneigt ist, während die abgewandte Rückfläche 8 zur Basisfläche einen Winkels von ca. 40° bildet. Die Höhe der Rückfläche 8 beträgt etwa 1,8 mm und die Gesamtlänge des Stents etwa 7 mm. Der Biegungsradius ist entsprechend der Krümmung des Schlemm'schen Kanals 2 gewählt und beträgt etwa 7 mm. Am einen Ende ist der Stent 1 mit einem Plättchen 9 zum Fassen mittels einer Pinzette, Zange od. dgl. versehen.

Aus Fig. 3 erkennt man, wie durch die besondere Parallel-Orientierung der Lichtaustrittsfläche 5 zur Innenfläche des Stents 1 unter Zusammendrückung des Gewebes 10 des Trabekelwerkes vor dem Schlemm'schen Kanal eine Positionierung erzielt wird, bei der seitlich reflektiertes Licht oder auf die Rückseite des Schlemm'schen Kanals auftreffendes Licht des Laserkatheters sicher verhindert ist, so dass ausschließlich vor dem Schlemm'schen Kanal unter Wegbrennen des Gewebes eine Öffnung erzeugt wird, durch die das Kammerwasser in den Schlemm'schen Kanal gelangen kann, durch den es schließlich abgeführt wird.

Die Fig. 4 zeigt einen im Wesentlichen der Fig. 3 entsprechenden vergrößerten Teilausschnitt aus Fig. 2 mit einer abgewandelten, eine Justierausnehmung 11 umfassenden Faseranordnung 3. Die in entsprechender Weise wie in Fig. 3 gegenüber der gegenüberliegenden Seitenfläche 6 des Stents im Schlemm'schen Kanal kongruent ausgebildete also parallel zu dieser Seitenfläche 6 angeordnete Lichtaustrittsfläche 5 bildet hier den Boden der Justierausnehmung 11, die den Stent oben und unten umgreift.

## Patentansprüche

1. Vorrichtung zur Glaukombehandlung mit einem Laserkatheter und einer lichtführenden Faseranordnung, an deren proximalem Ende Licht einkoppelbar ist und an deren distalem Ende eine Lichtaustrittsfläche vorgesehen ist, die bei der Behandlung einem im Schlemm'schen Kanal angeordneten gebogenen Stent gegenübersteht, **dadurch gekennzeichnet, dass** der Stent (1) einen im Wesentlichen dreieckigen Querschnitt derart aufweist, dass die Lichtaustrittsfläche (5) im Wesentlichen kongruent zur gegenüberliegenden Seitenfläche (6) des Stents (1) im Schlemm'schen Kanal (2) ausgebildet ist.

2. Vorrichtung nach Anspruch 1, **dadurch gekennzeichnet, dass** die Lichtaustrittsfläche (5) den Boden einer Justierausnehmung (11) der Faseranordnung (3) bildet, die den Stent (1) oben und unten umgreift.

3. Vorrichtung nach Anspruch 1 oder 2, **dadurch gekennzeichnet, dass** die auf der Bogeninnenseite liegende, der Lichtaustrittsfläche (5) des Laserkatheters (3) gegenüberliegende Innen-Seitenfläche (6) einen Winkel von ca. 115° mit der schmalen Basisfläche (7) und die auf der Bogenaußenseite liegende Außenfläche einen Winkel von ca. 40° mit der Basisfläche (7) des Stents (1) bilden.

4. Vorrichtung nach einem der Ansprüche 1 bis 3, **dadurch gekennzeichnet, dass** der Stent (1) einen Biegeradius von ca. 7 mm aufweist.

5. Vorrichtung nach einem der Ansprüche 1 bis 4, **dadurch gekennzeichnet, dass** am einen Ende des Stents (1) ein Plättchen oder eine Nocke (9) zum Fassen mittels einer Pinzette, Zange od. dgl. befestigt ist.
